# EUROPEAN PATENT APPLICATION

(11) **EP 2 462 980 A1**
(43) Date of publication of application: **13.06.2012**
(21) Application number: 10194149.0
(22) Date of filing: 08.12.2010
(51) Int. Cl.: A61M 39/20

(54) **Priming cap for an infusion system**

(71) Applicant: Unomedical A/S, 3460 Birkerød (DK)
(72) Inventor: Dirac, Holger, 3460, Birkerød (DK); Gundberg, Tomas, 4130, Viby Sj (DK); Frederiksen, John, Myhre, 4140, Borup (DK)
(74) Representative: Hansen, Anders Sandgaard

(57) **Abstract**

Disclosed is a priming cap (2) for an infusion system and a method for priming. The priming cap comprises a body member with a first end and a second end and having a wall with an inner and outer surface, the body member defining a first cavity, wherein the priming cap comprises a venting member allowing passage of air from the first cavity through the venting member and limiting passage of liquid from the first cavity through the venting member.

## Description

The present invention relates to a priming cap, in particular for an infusion system and method for priming an infusion system.

An infusion set of an infusion system may have a relatively long flow channel typically filled with air or gaseous fluid prior to use. During use, air is not desired in the flow channel since injection of air is not desirable and because air in the infusion set can lead to inaccurate amounts of medicine being administered. Accordingly, the infusion system requires priming, i.e. the flow channel of the infusion system requires filling with liquid or medicine before use.

Known methods for priming require visual inspection of the infusion set.

There is a need for devices and methods allowing easy and convenient priming of infusion systems.

### SUMMARY

The present invention enables convenient and easy priming of infusion systems.

Accordingly, a priming cap for an infusion system is provided, the priming cap extending along a first axis and comprising a body member with a first end and a second end and having a wall with an inner and outer surface, the body member defining a first cavity. The priming cap comprises a venting member allowing passage of air from the first cavity through the venting member and limiting passage of liquid from the first cavity through the venting member.

Further, a method for priming an infusion system is provided, the infusion system comprising a priming cap comprising a body member having a wall with an inner and outer surface, the body member defining a first cavity, wherein the priming cap comprises a venting member allowing passage of air from the first cavity through the venting member and limiting passage of liquid from the first cavity through the venting member, the infusion system comprising an infusion set having a flow channel with an output end. The method comprises pumping fluid through the flow channel towards the output end and into the first cavity of the priming cap, allowing passage of air in the fluid through the venting member, and limiting passage of liquid in the fluid through the venting member. Further, the method may comprise monitoring at least one operating parameter including a first operating parameter of the flow channel and transmitting a priming signal when a priming criterion based on the at least one operating parameter is fulfilled.

It is an important advantage of the present invention that a user with impaired body function, e.g. vision, is enabled to safely prime an infusion system. The present invention may also allow for reduced medicine spillage when priming an infusion system.

### BRIEF DESCRIPTION OF THE DRAWINGS

The above and other features and advantages of the present invention will become readily apparent to those skilled in the art by the following detailed description of exemplary embodiments thereof with reference to the attached drawings, in which:
- Fig. 1: schematically illustrates an exemplary priming cap,
- Fig. 2: schematically illustrates an exemplary priming cap ,
- Fig. 3: schematically illustrates an exemplary priming cap,
- Fig. 4: schematically illustrates an exemplary priming cap ,
- Fig. 5: schematically illustrates an exemplary priming cap,
- Fig. 6: schematically illustrates an exemplary priming cap,
- Fig. 7: shows a part of an infusion set with a priming cap,
- Fig. 8: shows a part of an infusion set with a priming cap,
- Fig. 9: shows a part of an infusion set with a priming cap,
- Fig. 10: shows a part of an infusion set with a priming cap, and
- Fig. 11: is a flow diagram of an exemplary method

### DETAILED DESCRIPTION

The figures are schematic and simplified for clarity, and they merely show details which are essential to the understanding of the invention, while other details have been left out. Throughout, the same reference numerals are used for identical or corresponding parts.

The priming cap comprises a body member. The body member has a wall, e.g. a tubular wall, defining at least one cavity including a first cavity. The wall may have a first opening at the first end of the body member and a second opening at the second end of the body member. The wall may have one or more side openings including a first side opening. The one or more side openings may function as pressure equalisation means when the priming cap is removed from the infusion set after or during priming of the infusion set.

During use, an output end of an infusion set may be accommodated in the first cavity. The body wall may be made of a suitable plastic material, such as a heat-shrinkable plastic material.

The priming cap comprises a venting member. The venting member may be positioned in the body member between the first cavity and the outside of the body member, i.e. at an end of the body member. The venting may be positioned between two cavities, e.g. between the first cavity and a second cavity, separating the two cavities.

The venting member, at least in a first state, may allow passage of air though the venting member. The venting member, at least in a second state, may limit or prevent passage of liquid from the first cavity through the venting member. The venting member may in the second state limit or prevent passage of air through the venting member. The venting member may in the first state limit or prevent passage of liquid from the first cavity through the venting member.

The venting member may comprise a micro-porous membrane allowing air, but not fluid to pass. Such micro-porous membrane may be made of a hydrophobic material, e.g. supported by a web or mesh material. The hydrophobic material may be polytetrafluorethylene (PTFE), such as expanded polytetrafluorethylene (ePTFE) known as Gore-Tex or Softform. A micro-porous membrane may limit passage of liquid, e.g. by having a liquid flow resistance larger than or at least a first threshold value. A micro-porous membrane may allow passage of air, e.g. by having an air flow resistance less than a second threshold value. The first threshold value is larger than the second threshold value.

The venting member may comprise a swellable material that in a first state or dry state allows passage of air. The swellable material may in a second state or wet state be swelled thereby limiting or blocking passage of liquid and optionally air through the venting member. A swellable material is a material that changes size upon contact with a liquid. The venting member may be made of a swelling agent, optionally in combination with a mesh material.

The mesh material may be made of a polymer, such as polyethylene, e.g. high density polyethylene (HDPE), polypropylene, polystyrene or the like.

Suitable swelling agents may be selected from cellulose polymers, such as carboxymethylecellulose (CMC), methyl-ethyl-cellulose or the like. Vinyl esters and/or acrylic and metacrylic acid esters may be a suitable swelling agent.

Swelling agents can also be homo- and copolymers of vinyl esters, in particular vinyl acetate homopolymers and vinyl acetat copolymers with ethylene, acrylates, maleic acid esters, vinylamides and/or other vinylacyl derivatives.

Swelling agents based on acrylic and methacrylic adic esters are homo- and copolymers for example copolymers of methyl methacrylate and n-butyl acrylate of 2-ethylhexyl acrylate. The swelling agent may comprise a co-monomer based on vinyl esters and acrylic and methacrylic acid esters, such as styrene, butadiene, vinylamides, olefinically unsaturated carboxylic acids and derivatives thereof, olefinically unsaturated sulphonic acids and vinylphosphonic acid or polyglycol esters of unsaturated acids.

Water-swellable hydrophilic polymers which can be used as swelling agents in a venting member include but are not limited to polyvinylpyrrolidone, polyvinyl alcohol, polyacrylic acid, polymethacrylic acid, polyacrylic amides. The swelling agent(s) may comprise or more of polymethacryl amides, and grafts or copolymer of any of these polymers e.g. copolymers with maleric anhydride (such as poly(methyl vinyl ether/maleric anhydride)), succinic anhydride or the corresponding acids, as well as polyamides, polyethylene glycols (PEG), gelatin, polysaccharides (e.g. cellulose derivatives such as carbolymethylcellulose, cellulose acetate, and cellulose acetate propionate, and chitosan), hydrophilic polyurethanes, and carbolylated butadiene styrene rubbers.

The venting member may comprise carriers, such as pellets, mesh or fibers, coated with one or more swelling agents and/or a liquid-activated adhesive.

The venting member may comprise pellets made of or coated with one or more swelling agents and/or a liquid-activated adhesive.

The venting member may comprise a one or more passage elements, e.g. made of liquid-soluble material, for allowing passage of liquid in a third state of the venting member from the first cavity through the venting member. A passage element may constitute the venting member. A passage element may be embedded in or mixed with a swellable material.

The venting member may have a flow resistance with a liquid flow profile, such that the liquid flow resistance of the venting member is larger than a first threshold value. The liquid flow resistance of the venting member may be selected such that a pump device pumping fluid through the flow channel and into the first cavity builds up a pressure in the cavity due to the large liquid flow resistance which leads to a limited liquid flow through the venting member. At the same time, the air flow resistance of the venting member may be selected less than a second threshold value such that air may flow through the venting member without building pressure in the flow channel larger than the pressure threshold of the pump device. When the pressure in the flow channel reaches a pressure threshold, pumping is stopped and a priming signal may be transmitted to the user indicating that priming is done.

The priming cap and method according to the invention allows for automatic priming of infusion sets by using the built-in occlusion alarm of a pump system. When an occlusion has been detected, the pressure in the flow channel is higher than the pressure in the surroundings leading to sprinkling of medicine or injection fluid when the priming cap is removed.

When the priming cap is attached to the infusion set, the output end of the infusion set is accommodated in the first cavity, and air flowing out of the output end can exit the first cavity via or through the venting member. Liquid flowing out of the output end is not able to or is restricted from exiting the first cavity resulting in accumulation of pressure in the flow channel above a pressure threshold or flow rates below a threshold.

In order to avoid sprinkling of medicine, the priming cap may comprise at least one absorption member including a first absorption member. The first absorption member may be positioned in the first cavity between the venting member and the first end of the body member or in a second cavity formed by the wall between the venting member and the second end of the body member. An absorption member, e.g. the first absorption member, may be positioned on the outside of the wall. The first absorption member may be slidably arranged in relation to the body member. A second absorption member may be positioned in the first cavity between the venting member and the first end of the body member or in a second cavity formed by the wall between the venting member and the second end of the body member. An absorption member may be arranged in a cavity of a body member sleeve or on the outside of a body member sleeve.

The wall of the body member may have a first region with a first inner cross-sectional diameter perpendicular to the first axis and may have a second region with a second inner cross-sectional diameter perpendicular to the first axis, the first region and the second region at least partly forming the first cavity. The second inner cross-sectional diameter may be greater than the first inner cross-sectional diameter. A transition region may be provided between the first and second regions along the first axis.

The first inner cross-sectional diameter of the wall may be in the range from about 0.1 mm to about 2 mm, e.g. about 1.3 mm. A first inner cross-sectional diameter between 0.4 mm and 0.8 mm, e.g. about 0.65 mm may be preferred for infusion sets.

The first inner cross-sectional diameter of the wall may substantially correspond to the outer diameter of the element forming the output end of the infusion set (e.g. cannula or needle).

The first region may have a first length (extension along the first axis). The length of the first region is selected in order to provide suitable frictional resistance between the priming cap and the infusion set part with the output end (e.g. a cannula and/or needle) during use. The first length may be at least 0.5 mm, such as about 1 mm about 2 mm, about 3 mm, or about 4 mm. A first length in the range from 1 mm to about 10 mm may be preferred.

The dimensions of the first region may be selected such that leakage of fluid between the wall and the infusion set (cannula and/or needle) is prevented or minimised. Thereby a sealing is formed between the wall and the infusion set (cannula and/or needle) such that the venting member is the only passageway to the surroundings for fluid flowing out of the infusion set during priming.

The second inner cross-sectional diameter of the wall may be in the range from about 0.5 mm to about 6 mm, such as in the range from about 1.0 mm to about 4 mm, e.g. about 1.5 mm.

The second region may have a second length (extension along the first axis). The length of the second region is selected in order to provide suitable volume of the first cavity. The second inner cross-sectional diameter may vary in the second region. The second length may be at least 1 mm, such as about 2 mm, about 3 mm, about 4 mm or about 5 mm. A second length in the range from 2 mm to about 10 mm may be preferred.

The difference between the (maximum) first inner cross-sectional diameter and the (minimum) second inner cross-sectional diameter of the wall may be at least 0.3 mm, such as at least 0.5 mm, e.g. about 0.8 mm. A large difference may be desirable in order to increase the volume for accommodation of spillage. A large second inner cross-sectional diameter of the wall may however reduce retention of liquid in the priming cap.

The priming cap may comprise a body member sleeve slidably arranged in relation to the body member along the first axis. The body member sleeve may in a first position accommodate a main part of the body member and may in a second position be able to accommodate liquid that flows out of the output end of the infusion set.

The body member sleeve may be slidably arranged on the outside of the body, i.e. the body member sleeve may be an outer body member sleeve, and/or the body member sleeve may be slidably arranged within the body member, i.e. the body member sleeve may be an inner body member sleeve. The body member sleeve may have a first end and a second end and comprising a wall. The body member sleeve may, e.g. at one or both ends of the body member sleeve, comprise an outwardly and/or inwardly extending flange or protrusion, e.g. in order to provide a stop member and/or sealing with the body member and/or a sealing with an infusion set.

An outer body member sleeve may have an inner cross-sectional diameter substantially corresponding to the largest outer diameter of the body member along a sliding region (the part of body member accommodated within outer body member sleeve), e.g. the outer diameter of an outwardly extending flange at the first end of the body member.

An inner body member sleeve may have an outer cross-sectional diameter substantially corresponding to the smallest inner cross-sectional diameter of the body member along a sliding region (the part of the inner body member sleeve accommodated within body member), e.g. the inner diameter of an inwardly extending flange at the first end of the body member.

The body member may at one or both ends comprise an outwardly and/or inwardly extending flange, e.g. in order to provide a stop member and/or sealing with a body member sleeve and/or a sealing with the infusion set.

In order to reduce sprinkling of liquid during or after priming, an infusion system comprising a priming cap and an absorption member comprising a liquid-impermeable base layer and at least a first absorption layer is provided.

The method for priming an infusion system comprises monitoring at least one operating parameter of the flow channel. A first operating parameter may be the pressure in the flow channel. Other monitored operating parameters may include one or more of flow volume, speed and/or acceleration.

In the method, a priming signal is transmitted when a priming criterion is fulfilled.

The priming criterion may be whether an operating parameter is greater than or less than a threshold value. The priming criterion may depend on one or more operating parameters as a function of time. The priming criterion may be whether the pressure in the flow channel is greater than a pressure threshold value.

The method may comprise removing the priming cap from the infusion set when the priming signal has been transmitted. The method may comprise reducing the pressure in the flow channel when the priming criterion is fulfilled, e.g prior to or by removing the priming cap.

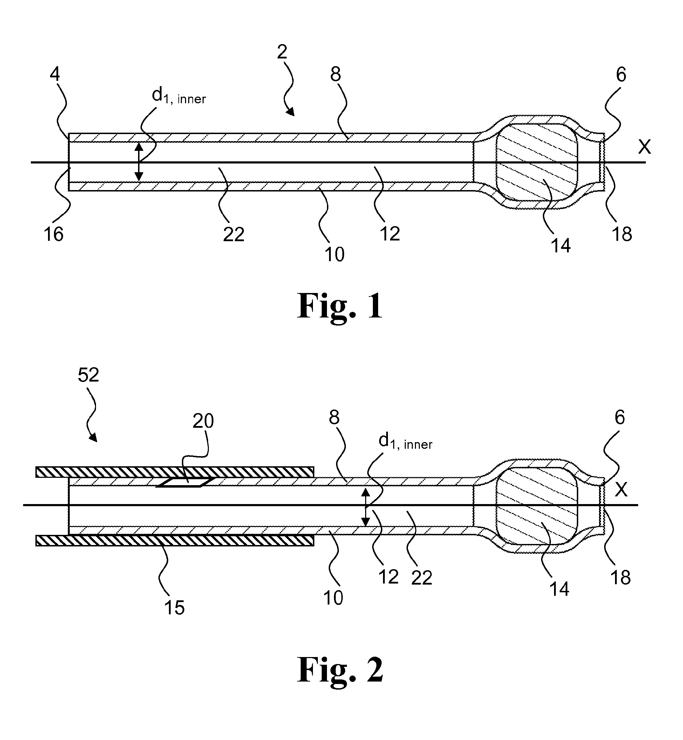

Fig. 1 is a cross-sectional view of an exemplary priming cap for an infusion system having a flow channel. The priming cap 2 extends along a first axis X and comprises a body member 8 with a first end 4 and a second end 6 and having a wall 10 with an inner and outer surface, the body member 8 defining a first cavity 12. The priming cap 2 comprises a venting member 14 allowing, at least in a first state, passage of air from the first cavity 12 through the venting member 14. Further, the venting member 14 limits passage of liquid from the first cavity 12 through the venting member 14.

The venting member 14 illustrated in Fig. 1 comprises a swellable material such as fiber material made of or coated with a swellable material. In a first state when the venting member 14 is dry, the venting member allows air to flow through the venting member such that the operating pressure P₁ in the first cavity and in the flow channel is below a pressure threshold P₁, threshold. When a liquid, e.g. medicine or water, reaches the venting member 14, the swellable material of the venting member 14 swells and in a second state limits or blocks passage of liquid and air which leads to a change in one or more operating parameters of the flow channel of the infusion system, such as an increase of the pressure in the first cavity 12 and the flow channel of the infusion system, reduction in flow volume, speed or acceleration or other suitable operating parameters.

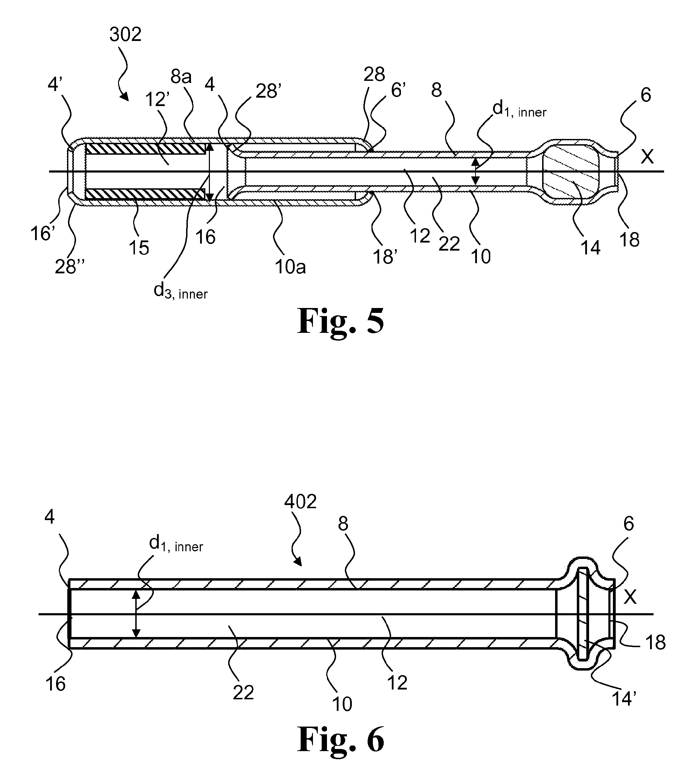

The venting member 14 may be adapted to allow passage of air in a state where passage of liquid is limited or blocked. For example, the venting member 14 may as illustrated in Fig. 6 comprise a hydrophobic membrane 14' optionally supported by a mesh or web.

Fig. 2 is a cross-sectional view of an exemplary priming cap 52. The priming cap 52 corresponds to the priming cap 2 with one or more optional features. The priming cap 52 may optionally comprise at least one absorption member 15. The absorption member 15 may extend beyond the first end 4 of the body member 8 in order to absorb liquid flowing out of the first opening 16 in the body member 8. The absorption member 15 of the priming cap 52 is an outer absorption member arranged on the outside of the body member 8. Alternatively or in combination with the outer absorption member, an inner absorption member may be provided in the first cavity 12. The absorption member 15 encloses a part of the body member 8.

The priming cap 52 optionally comprises one or more side openings including a first side opening 20 in the wall 8. A side opening forms a passage way from the first cavity 12 to the surroundings. During priming, the one or more side openings are blocked by the infusion set (cannula or needle). When the priming cap is removed from the infusion set, the pressure in the flow channel/first cavity is equalized through the one or more side openings. The absorption member 15 may cover the one or more side openings in order to absorb liquid flowing out of the side opening(s).

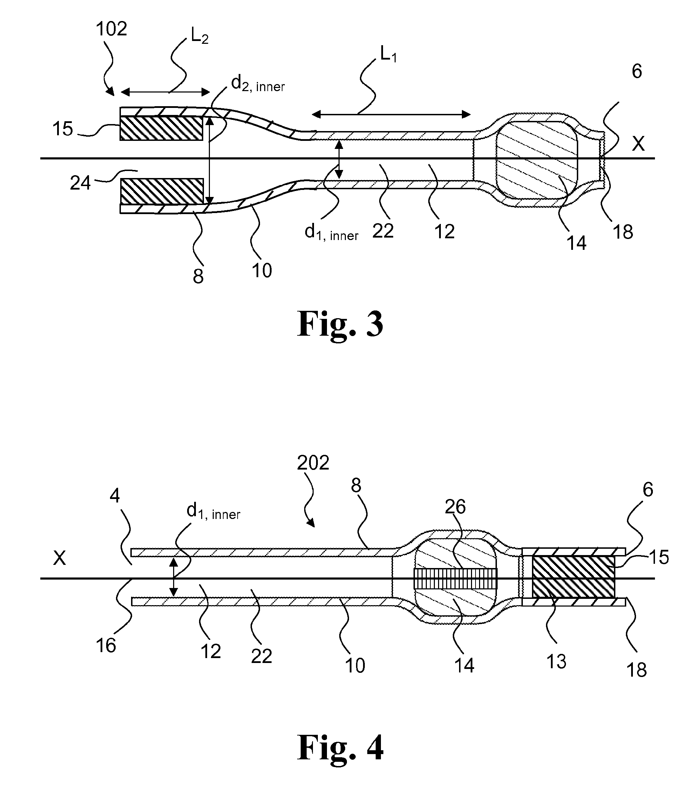

Fig. 3 is a cross-sectional view of an exemplary priming cap 102. The wall 10 of the body member 8 defines a first cavity with a first region 22 with a first inner cross-sectional diameter d₁, ᵢₙₙₑᵣ perpendicular to the first axis X. The first inner cross-sectional diameter d₁, ᵢₙₙₑᵣ of priming cap 102 is about 0.65 mm substantially corresponding to an outer diameter of cannula and/or needle of an infusion set. The first region 22 provides attachment of the priming cap 102 on the infusion set during priming. The first cavity 12 has a second region 24 with a second inner cross-sectional diameter d₂, inner perpendicular to the first axis X. The second inner cross-sectional diameter is greater than the first inner cross-sectional diameter in order to increase the volume of the first cavity to accommodate a suitable volume of liquid, e.g. such that the first cavity volume is large enough to accommodate at least a majority of the liquid flowing out of the output end of an infusion set during and immediately after priming when the pump device has stopped pumping. The second inner cross-sectional diameter of the second region 24 is about 1.5 mm. The priming cap optionally comprises an absorption member 15 within the first cavity in the second region.

The first region 22 has a first length L₁ (extension along the first axis). The first length is selected in order to provide suitable frictional resistance between the priming cap and the infusion set part with the output end (e.g. a cannula and/or needle) during priming. The first length L₁ is at least 2 mm. A first length in the range from 1 mm to about 10 mm is preferred.

The second region 24 has a second length L₂ (extension along the first axis). The second length L₂ may be in the range from 1 mm to 10 mm. The second length L₂ for the priming cap 102 is about 2 mm.

Fig. 4 is a cross-sectional view of an exemplary priming cap 202. The priming cap 202 comprises a venting member 14 comprising a passage element 26 made of liquid-soluble material. When exposed to liquid, the passage element 26 dissolves thereby forming a passageway from the first cavity through the venting member 14. When the passage element 26 is dissolved after time t_{dis} (venting member 14 in third state) liquid passes through the venting member 14 thereby enabling pressure equalization through the venting member in a third state. The priming cap 202 optionally comprises an absorption member 15 at least partly accommodated in a second cavity 13 formed by the wall 10 between the venting member 14 and the second end 6.

Fig. 5 is a cross-sectional view of an exemplary priming cap 302. The priming cap comprises a body member 8 and a body member sleeve 8a slidably arranged in relation to the body member 8 along the first axis X. The body member sleeve 8a has a wall 10a with a first end 4' and a second end 6' and defining a cavity 12'. The body member sleeve 8a is an outer body member sleeve where the cavity 12' in a first position as shown in Fig. 5 accommodates a main part of the body member 8.

Optionally, an absorption member 15 is arranged in the cavity 12' of the body member sleeve 8a. Upon priming, a user slides the body member 8 in relation to the body member sleeve 8a to a second position whereby the cavity 12' thereby forming a reservoir for liquid flowing out of the output end of the infusion set. Accordingly, in a second position, the priming cap 302 is able to accommodate liquid that flows out of the output end of the infusion set.

The body member sleeve 8a comprises an inwardly extending flange 28 with an opening 18' at the second end 6' thereby providing a stop and guide member for the body member 8. The diameter of the opening 18' corresponds to the outer diameter of the body member 8 thereby forming a sealing with the body member 8.

The body member sleeve 8a comprises an inwardly extending flange 28" with an opening 16" at the first end 4'. The diameter of the opening 16' corresponds to the outer diameter of cannula or needle of infusion set thereby providing a seal and guide between the body member sleeve and the infusion set.

The body member 8 comprises an outwardly extending flange 28' at the first end 4. The flange 28' has an outer diameter corresponding to the inner diameter d₃, inner of the body member sleeve 8a thereby forming a stop member and/or sealing with the body member sleeve.

Fig. 6 is a cross-sectional view of an exemplary priming cap 402. In the priming cap 402, the venting member 14' is a micro-porous hydrophobic membrane allowing passage of air and blocking or restricting passage of liquid through the venting member.

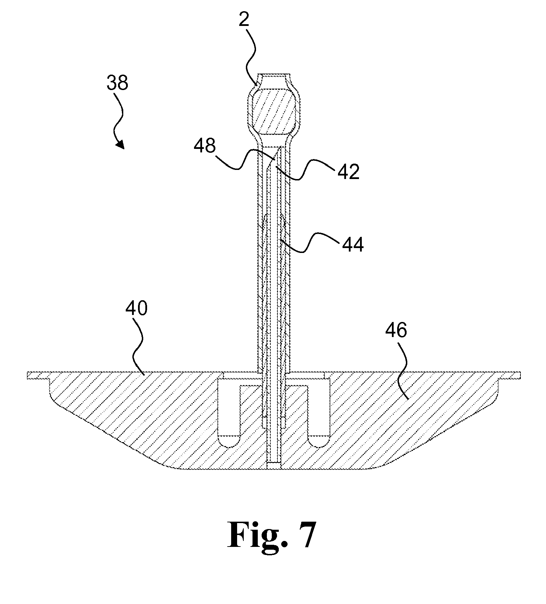

Fig. 7 shows a part of an infusion system comprising a part of an infusion set with a priming cap 2 attached. The infusion set 40 comprises an infusion needle 42 optionally enclosed by a cannula 44 mounted on a cannula base part 46. The needle 42 may be mounted to the cannula base part 46 or to a separate needle base part (not shown). The output end 48 of the infusion set 40 is positioned in the first cavity of the priming cap 2 that is attached to the cannula 44 by frictional forces also forming a seal between the wall 10 and the cannula 44. The infusion set may be connected to a pump device pumping fluid from a reservoir to the output end, e.g. via a tube of the infusion set.

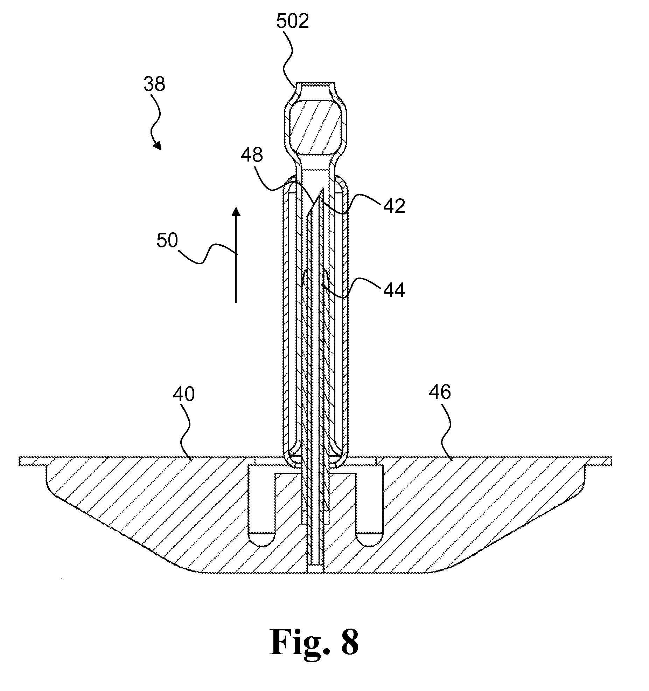

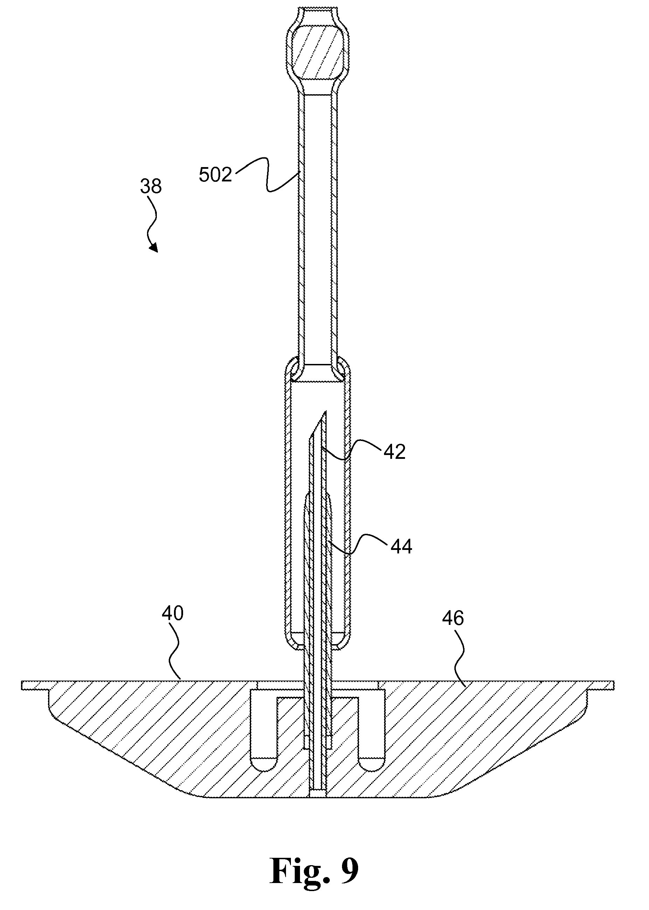

Fig. 8 and 9 shows a part of an infusion system comprising a part of an infusion set with a priming cap 502 attached in a first position and a second position, respectively. The priming cap 502 corresponds to the priming cap 302 but without the absorption member 15. When priming is done, i.e. a priming criterion is fulfilled, and pumping is stopped, the body member 8 is moved or pulled into the second position in the direction indicated by arrow 50 sliding the body member in relation to the body member sleeve and separating the body member from the cannula and needle. Upon separation of the body member and cannula, superfluous liquid flow into the cavities 12, 12' resulting from overpressure in the flow channel is able to flow into the cavity of the body member sleeve. In the second position, the two flanges 28 and 28' act as stop members preventing separation of the body member 8 and the body member sleeve 8a and optionally act as sealing members ensuring substantially liquid tight sealing between the body member 8 and the body member sleeve 8a. The cavity 12' of the body member sleeve (together with the first cavity 12 of the body member) accommodates liquid flowing out of the output end due to over-pressure in the flow channel after priming. The priming cap 502 is attached to the cannula 44 by frictional forces also forming a seal between the wall 10 of the priming cap 502 and the cannula 44. When the pressure in the flow channel has been reduced, the body member sleeve is removed from the infusion set which is then ready for insertion.

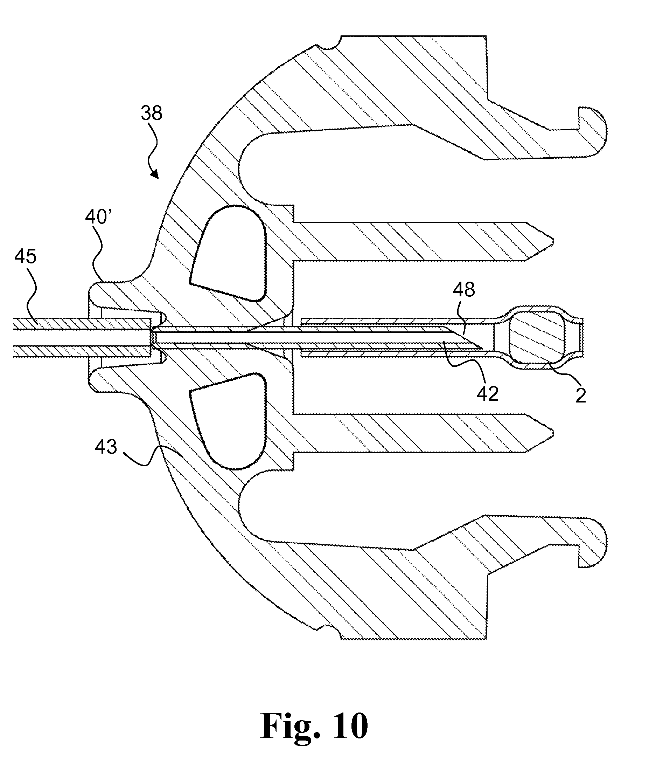

Fig. 10 shows a part of an infusion system comprising a part of an infusion set with a priming cap 2 attached. The infusion set of Fig. 10 may be connected to a part with a cannula or other subcutaneous element after priming. The infusion set 40' comprises a needle 42 mounted to a needle base part 43. A tube 45 is also mounted to the base part 43. The needle base part 43 may be adapted for locking or releasable coupling to a connector for connection to a cannula part. The output end 48 of the infusion set 40' is positioned in the first cavity of the priming cap 2 that is attached to the cannula 44 by frictional forces also forming a seal between the wall 10 and the cannula 44. The infusion set may be connected to a pump device pumping fluid from a reservoir to the output end, e.g. via a tube of the infusion set.

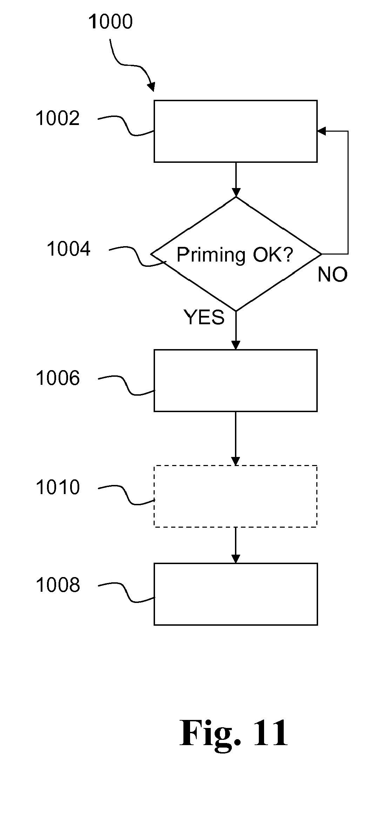

Fig. 11 is a flow diagram of a method for priming an infusion system comprising a priming cap, e.g. priming cap 2, 52, 102, 202, 302, 402, 502, comprising a body member having a wall with an inner and outer surface, the body member defining a first cavity, wherein the priming cap comprises a venting member allowing passage of air from the first cavity through the venting member and limiting passage of liquid from the first cavity through the venting member, the infusion system comprising an infusion set having a flow channel with an output end. The method 1000 comprises pumping 1002 fluid through the flow channel towards the output end and into the first cavity of the priming cap, e.g. with a pump device of the infusion system. Further, the method comprises allowing 1002 passage of air in the fluid through the venting member, and limiting 1002 passage of liquid in the fluid through the venting member. Further, the method comprises monitoring 1004 at least one operating parameter including a first operating parameter of the flow channel and transmitting 1006 a priming signal when a priming criterion based on the at least one operating parameter is fulfilled. The priming signal may be a sound signal, a light signal or a combination thereof. In the method 1000, the first operating parameter is the pressure in the flow channel and the priming criterion is whether the pressure in the flow channel is larger than a pressure threshold. Monitoring 1004 the pressure in the flow channel may be performed by a pump device. The method may comprise removing 1008 the priming cap from the infusion set when the priming signal has been transmitted. The method 1000 optionally (indicated by dotted box) comprises reducing 1010 the pressure in the flow channel when the priming criterion is fulfilled and before the priming cap is removed.

It should be noted that in addition to the exemplary embodiments of the invention shown in the accompanying drawings, the invention may be embodied in different forms and should not be construed as limited to the embodiments set forth herein. Rather, these embodiments are provided so that this disclosure will be thorough and complete, and will fully convey the concept of the invention to those skilled in the art.

### LIST OF REFERENCES

- 1: Infusion system
- 2, 52, 102, 202, 302, 402, 502: Priming cap
- 4, 4': First end
- 6, 6': Second end
- 8: Body member
- 8a: Body member sleeve
- 10: Wall
- 10a: Wall
- 12: First cavity
- 12': Cavity
- 13: Second cavity
- 14: Venting member
- 14': Hydrophobic membrane
- 15: Absorption member
- 16, 16': First opening
- 18, 18': Second opening
- 20: First side opening
- 22: First region
- 24: Second region
- 26: Passage element
- 28, 28', 28": Flange
- 38: Infusion system
- 40,40': Infusion set
- 42: Needle
- 43: Needle base part
- 44: Cannula
- 45: Infusion tube
- 46: Cannula base part
- 48: Output end
- 50: Direction of removal
- P₁, threshold: Pressure threshold
- P₁: Pressure
- d₁, inner: First inner cross-sectional diameter
- d₂, inner: Second inner cross-sectional diameter
- d₃, inner: inner diameter of body member sleeve
- L₁: First length
- L₂: Second length
- X: First axis

## Claims

1. Priming cap for an infusion system, the priming cap extending along a first axis and comprising a body member with a first end and a second end and having a wall with an inner and outer surface, the body member defining a first cavity, wherein the priming cap comprises a venting member allowing passage of air from the first cavity through the venting member and limiting passage of liquid from the first cavity through the venting member.

2. Priming cap according to claim 1, wherein the venting member in a first state allows passage of air from the first cavity through the venting member and in a second state limiting passage of liquid from the first cavity through the venting member.

3. Priming cap according to any of the preceding claims, wherein the venting member comprises a micro-porous hydrophobic membrane.

4. Priming cap according to any of the preceding claims, wherein the venting member comprises a swellable material that in a first state allows passage of air and in a second state is swelled thereby limiting or blocking passage of liquid.

5. Priming cap according to any of the preceding claims, wherein the venting member comprises material coated with a liquid-activated adhesive.

6. Priming cap according to any of the preceding claims, comprising at least one absorption member including a first absorption member.

7. Priming cap according to claim 6, wherein the first absorption member is slidably arranged in relation to the body member

8. Priming cap according to any of the preceding claims, wherein the wall has a first opening at the first end and a second opening at the second end.

9. Priming cap according to any of the preceding claims, wherein the wall has a first side opening.

10. Priming cap according to any of the preceding claims, wherein the wall has a first region with a first inner cross-sectional diameter perpendicular to the first axis and a second region with a second inner cross-sectional diameter perpendicular to the first axis, the first region and the second region at least partly forming the first cavity, wherein the second inner cross-sectional diameter is greater than the first inner cross-sectional diameter, and wherein the first inner cross-sectional diameter is in the range from about 0.1 mm to about 2 mm and the second inner cross-sectional diameter being in the range from about 0.5 mm to about 6 mm.

11. Priming cap according to any of the preceding claims, comprising a body member sleeve slidably arranged in relation to the body member along the first axis.

12. Priming cap according to any of the preceding claims, wherein the venting member comprises a passage element made of liquid-soluble material for allowing passage of liquid in a third state of the venting member from the first cavity through the venting member.

13. Priming cap according to any of the preceding claims, wherein the venting member has a flow resistance with a liquid flow profile, such that the flow resistance of the venting member is larger than a first threshold value.

14. Infusion system comprising a priming cap according to any of the preceding claims and an absorption member comprising a liquid-impermeable base layer and at least a first absorption layer.

15. Method for priming an infusion system comprising a priming cap comprising a body member having a wall with an inner and outer surface, the body member defining a first cavity, wherein the priming cap comprises a venting member allowing passage of air from the first cavity through the venting member and limiting passage of liquid from the first cavity through the venting member, the infusion system comprising an infusion set having a flow channel with an output end, wherein the method comprising:
- pumping fluid through the flow channel towards the output end and into the first cavity of the priming cap,
- allowing passage of air in the fluid through the venting member,
- limiting passage of liquid in the fluid through the venting member;
- monitoring at least one operating parameter including a first operating parameter of the flow channel; and
- transmitting a priming signal when a priming criterion based on the at least one operating parameter is fulfilled.
